# EUROPEAN PATENT APPLICATION

(11) **EP 3 399 362 A1**
(43) Date of publication of application: **07.11.2018**
(21) Application number: 15911818.1
(22) Date of filing: 30.12.2015
(51) Int. Cl.: G02B 27/01

(54) **HEAD-MOUNTED DISPLAY APPARATUS**

(71) Applicant: Shenzhen Royole Technologies Co., Ltd., Shenzhen, Guangdong 518172 (CN)
(72) Inventor: SHI, Hongyan, Shenzhen, Guangdong 518052 (CN)
(74) Representative: Hamer, Christopher K.
(86) International application number: PCT/CN2015/099869
(87) International publication number: WO 2017/113189

(57) **Abstract**

The present disclosure discloses a head-mounted display device. The present disclosure discloses a head-mounted display device comprising a display module, a diopter detection module, a diopter adjustment module and a controller. The display module is configured to project display images to exit pupil along a preset light path. The diopter detection module is configured to detect a parameter of a direction of exit pupil reflecting a diopter. The diopter adjustment module is configured to determine whether or not the diopter standard threshold is met according to the parameter detected by the diopter detection module. If not, the controller controls the diopter adjustment module to adjust, and further continuously acquires a current parameter detected by the diopter detection module during adjustment of the diopter adjustment module, until the current parameter detected by the diopter detection module is determined to meet the diopter standard threshold. The head-mounted display device detects the diopter of the wearer's eye, and automatically perform diopter correction when the diopter of the wearer's eye is incorrect, and is suitable for use by different people.

## Description

### TECHNICAL FIELD

This disclosure relates to head mounted displaying fields, and more particularly relates to a head mounted display device.

### BACKGROUND

With the development of science and technology, the head mounted display devices have gradually become civilian, which provide more and more people with high quality visual experiences. In order to meet the needs of the users with nearsightedness or farsightedness, the existing head mounted display devices are required to be able to correct the user's eyes. At present, there are two types of treatment ways for nearsightedness or farsightedness: the first way is to wear the corrective glasses and then wear the head mounted display device. However, the disadvantage of this way is that there is an oppression sense during wearing, which causes uncomfortable. The second way relies on adjusting the diopter manually. The head mounted display device usually includes a magnifying lens that can change the focal length, and the user can manually correct the eye by changing the focal length of the magnifying lens and changing the working distance of the lens. However, the disadvantage of the second way is that it is difficult for non-professionals to adjust accurately to match their own diopters, resulting in damaging the eyes while prolonged wearing the equipment. In addition, the manual adjustment process of the second way is cumbersome, and the design of the imaging lens determines the diopter adjustment range. Once the design is determined, the adjustment range is not variable and inflexible, and the adjustment range is limited by the lens design and space design.

### SUMMARY

Embodiments of the present disclosure disclose a head-mounted display device capable of automatically detecting a diopter of a wearer's eye and determining whether or not the diopter standard threshold is met according to the detected parameter. If not, the diopter adjustment module is controlled to adjust so as to correct the diopter of the wearer's eye to be normal, and suitable for different people.

Embodiments of the present disclosure disclose a head-mounted display device comprises a display module configured to project the display images to the exit pupil direction in a preset light path. The head-mounted display device also comprises a diopter detection module, a diopter adjustment module and a controller. The diopter detection module is configured to detect a parameter of the direction of exit pupil reflecting a diopter. The diopter adjustment module is configured to determine whether or not the diopter standard threshold is met according to the parameter detected by the diopter detection module. When the diopter standard threshold is not met, the controller controls the diopter adjustment module to adjust, and further continuously acquires a current parameter detected by the diopter detection module during adjustment of the diopter adjustment module, until the current parameter detected by the diopter detection module is determined to meet the diopter standard threshold.

The head-mounted display device in accordance with embodiments of the present disclosure can automatically detect the diopter of the wearer's eye and detect the parameter to determine whether it meets the diopter standard threshold. If not, the diopter adjustment module is controlled to adjust the diopter of the wearer's eye, so as to correct the diopter of the wearer's eye to be normal and suitable for different people.

### BRIEF DESCRIPTION OF THE DRAWINGS

To describe the technology solutions in the embodiments of the present disclosure more clearly, the following briefly introduces the accompanying drawings required for describing the embodiments. Obviously, the accompanying drawings in the following description show merely some embodiments of the present disclosure, those of ordinary skill in the art may also derive other obvious variations based on these accompanying drawings without creative efforts.
FIG. 1 is a block diagram of a head mounted display device in accordance with an embodiment of the present disclosure;
FIG. 2 is a schematic diagram of the head mounted display device shown in FIG. 1;

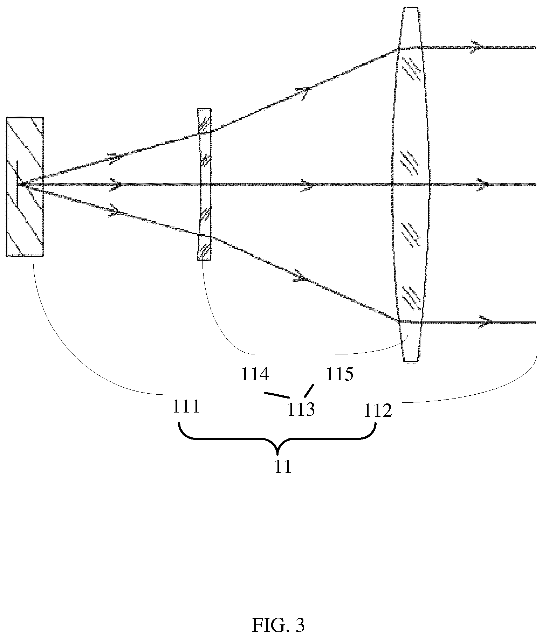

FIG. 3 is a schematic diagram of a transmitter module of the head mounted display device shown in FIG. 1;
FIG. 4 is a schematic diagram of a receive module of the head mounted display device shown in FIG. 1.

### DETAILED DESCRIPTION

The technical solution in the embodiments of the present disclosure will be described clearly and completely hereinafter with reference to the accompanying drawings in the embodiments of the present disclosure. Obviously, the described embodiments are merely some but not all embodiments of the present disclosure. All other embodiments obtained by a person of ordinary skilled in the art based on the embodiments of the present disclosure without creative efforts shall fall within the protection scope of the present disclosure.

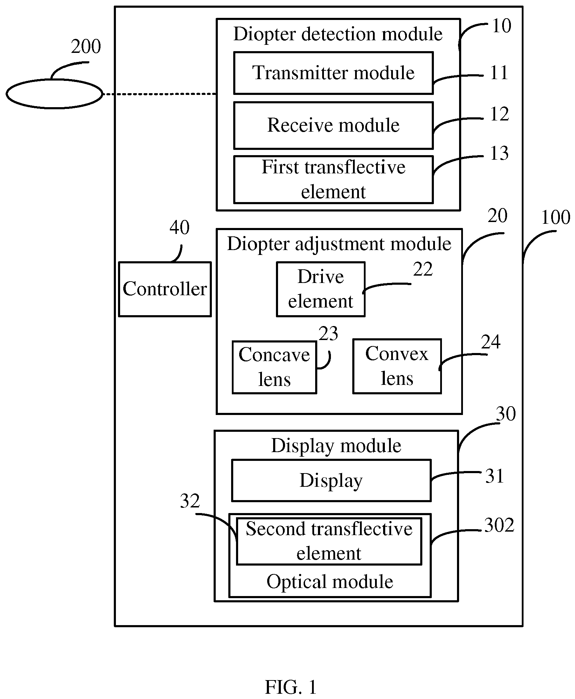

Referring to FIG. 1, FIG. 1 is a block diagram of a head mounted display device 100 in accordance with an embodiment of the present disclosure. The head mounted display device 100 includes a diopter detection module 10, a diopter adjustment module 20, a display module 30 and a controller 40. The display module 30 is configured to project display images to a direction of exit pupil. When the wearer wears the head mounted display device 100, the wearer's eye 200 is corresponded to the optical exit pupil of the display module 30 of the wearable display device. In order to illustrate the solution of the present disclosure, the following will indicate the exit pupil with the wearer's eye 200. The diopter detection module 10 is configured to detect a parameter of the direction of exit pupil reflecting the diopter of the wearer's eye 200. The controller 40 is configured to determine whether or not the parameter meets the diopter standard threshold according to the parameter detected by the diopter detection module 10. That is, the controller 40 is configured to determine whether or not the diopter of the wearer's eye 200 is normal. And when it is determined that the parameter does not meet the diopter standard threshold, that is, it is determined that the diopter of the wearer's eye 200 is abnormal, the controller 40 is configured to control the diopter adjustment module 20 to adjust the focal length of the preset light path, and further continuously acquire the current parameter detected by the diopter detection module 10 during adjustment of the diopter adjustment module 20, until the current parameter detected by the diopter detection module 10 is determined to meet the diopter standard threshold, that is, the wearer's eye 200 is corrected to be normal. The display module 30 is configured to generate display images for the wearer watching when the diopter is adjusted to be normal by the diopter adjustment module 20.

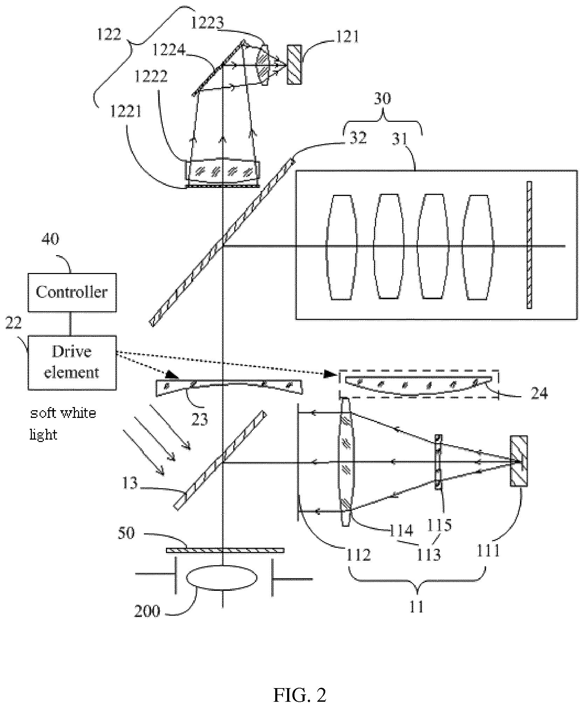

Please referring to FIG. 2, FIG. 2 is a specific structure diagram of the head mounted display device 100 in accordance with an embodiment of the present disclosure. The diopter detection module 10 includes a transmitter module 11 and a receive module 12. Therein, the transmitter module 11 is configured to transmit a near-infrared light to the direction of exit pupil of the head mounted display device 10, that is, the near-infrared light is projected to the wearer's eye 200 when the head mounted display device 100 is worn by the wearer. The receive module 12 is configured to receive the near-infrared light reflected by the direction of exit pupil, that is, the near-infrared light reflected by the retina of the wearer's eye 200 according to the near-infrared light of the transmitter module 11. The near-infrared light received by the receiving module 12 is the parameter reflecting the diopter of the wearer's eye.

The diopter adjustment module 20 includes a drive element 22, a concave lens 23 and a convex lens 24. The controller 40 is configured to determine whether or not it is necessary to correct the diopter of the wearer's eye according to the near-infrared light received by the receive module 12. The drive element 22 is controlled to drive the concave lens 23 or the convex lens 24 to act on the optical path accordingly when it is determined that it is necessary to correct the diopter of the wearer's eye, for example, it is located directly in front of the wearer's eye. The controller 40 controls the concave lens 23 or the convex lens 24 for focus adjusting correspondingly, for example, moving closer to or moving away from the wearer's eye, until the diopter of the wearer's eye is determined to be normal according to the near-infrared light reflected by the wearer's eye retina and received by the receive module 12.

In this embodiment, the transmitter module 11 includes a near-infrared light transmitter 111 and a small aperture stop 112. The near-infrared light transmitter 111 is configured to generate a near-infrared light and form a near-infrared aperture spot through the small aperture stop 112 and project it to the wearer's eye 200. The receive module 12 is configured to receive the near-infrared aperture spot reflected by the wearer's eye retina.

In detail, as shown in FIG. 2, in this embodiment, the near-infrared light transmitter 111 is arranged on the side of the sight line for watching of the wearer's eye 200. The diopter detection module 10 further includes a first transflective element 13. When the head mounted display device 100 is worn, the first transflective element 13 is located between the wearer's eye and the receive module 12. And the reflection plane of the first transflective element 13 and the direction of the near-infrared light generated by the transmitter module 11 are opposite to each other and are at an angle. The first transflective element 13 is configured to reflect the near-infrared light transmitted by the transmitter module 11 to the wearer's eye, and allow the near-infrared light reflected by the wearer's eye retina to be transmitted to the receiving module 12 .

The near-infrared light generated by the near-infrared light transmitter 111 passes through the small aperture stop 112 to form the near-infrared aperture spot. The near-infrared aperture spot is projected onto the first transflective element 13 and is reflected by the first transflective element 13 to the wearer's eye. Therein, the small aperture stop 112 is a light insulating barrier with a through hole in the middle thereof. The near-infrared light can only pass through the middle through hole, and the near-infrared light transmitted by the near-infrared light transmitter 111 passes through the small aperture stop 112 to form the near-infrared aperture spot. Therein, in this embodiment, the diameter of the through hole of the small aperture stop is 0.1mm. Therein, in at least one embodiment, the transmitter module 11 and the receive module 12 are at the same side, that is, the side which is right facing the wearer's eye 200, and the optical propagation paths are multiplexed by time division multiplexing, and the first transflective element 13 can be omitted.

The near-infrared aperture spot reflected by the retina of the wearer's eye 200 passes through the first transflective element 13 and is transmitted to the receive module 12. The receive module 12 includes a near-infrared receiver 121 for receiving the near-infrared aperture spot reflected by the wearer's retina. More precisely, the near-infrared aperture spot received by the receive module 12 is the parameter that reflects the diopter of the wearer's eye.

The controller 40 determines whether or not the wearer's eye 200 is a normal eye, nearsightedness or farsightedness, based on the size of the near-infrared aperture spot received by the near-infrared receiver 121, that is, it is determined whether or not the diopter of the wearer's eye is normal. The controller 40 determines that the wearer's eye is the normal eye when the diameter of the spot received by the near-infrared receiver 121 is 0.1 mm (millimeters), that is, it is not necessary to perform diopter correction for the wearer's eye. The spot with the diameter of 0.1 mm is the standard diopter threshold. When the diameter of the spot received by the near-infrared receiver 121 is not equal to 0.1 mm, the controller 40 determines that the wearer's eye is abnormal, that is, nearsightedness or farsightedness, and requires a diopter correction. In detail, the controller 40 determines that the wearer's eye is farsightedness when the diameter of the spot received by the receive module 12 is greater than 0.1mm, and determines that the wearer's eye is nearsightedness when the diameter of the spot received by the receive module 12 is less than 0.1mm.

Therein, the near-infrared receiver 121 can be a focus detector, a CCD (Charge Coupled Device). The near-infrared receiver 121 acquires the position projected by the near-infrared ray which is reflected by the retina of the wearer's eye 200, and the controller 40 calculates the distance between the position and the reference center, that is, the spot radius, which is multiplied by 2 to obtain the diameter of the spot.

Therein, the drive element 22 is a small-sized motor, such as an ultrasonic motor. The wearable display device 100 may be a wearable smart glasses. The initial state of the concave lens 23 and the convex lens 24 is rotated to a non-optical path position of the wearable display device 100, for example, located near the upper frame, and does not act on the optical path. When the controller 40 determines that the diopter correction of the wearer's eye is required and the wearer's eye is nearsightedness, the drive element 22 is controlled to drive the concave lens 23 to rotate to be directly in front of the wearer's eye, and the drive element 22 is controlled to drive the concave lens 23 to move relative to the wearer's eye until it is determined that the spot diameter received by the receive module 12 is equal to 0.1 mm.

When the controller 40 determines that the wearer's eye is farsightedness, the drive element 22 is controlled to drive the convex lens 24 to rotate to be directly in front of the wearer's eye, and the drive element 22 is controlled to drive the concave lens 24 to move relative to the wearer's eye until it is determined the spot diameter received by the receive module 12 is equal to 0.1 mm.

Therein, during the movement of the concave lens 23 or the convex lens 24, the size of the near-infrared aperture spot received by the receive module 12 also changes in real time. The controller 40 obtains the near-infrared aperture spot received by the receive module 12 in real time, and determines whether or not the current diameter of the near-infrared aperture spot is equal to 0.1 mm. If it is not equal, the concave lens 23 or the convex lens 24 is further controlled to move closer to or away from the wearer's eye until the diameter of the current near-infrared aperture spot is equal to 0.1 mm.

Therein, the concave lens 23 and the convex lens 24 can be mounted on a shaft (not shown) parallel to the wearer's sight line and can be rotated relative to the shaft under the drive of the drive element 22 so as to be located directly in front of the wearer's eye (ie, corresponding to the position of the pupil). The concave lens 23 and the convex lens 24 can be acted on an inactive position of the optical path as a corrective element, or rotate to be outside the sight line of the wearer's eye so as not to act on the optical path. The concave lens 23 and the convex lens 24 can move along the axis so as to move closer to or away from the wearer's eye under the drive of the drive element 22.

Therein, both sides of the concave lens 23 and the convex lens 24 are plated with a visible light near-infrared broadband antireflective material.

As shown in FIG. 2, in this embodiment, after the first transflective element 13 can also be set with soft natural white light irradiation to make the human eye in a relaxed state, so as to reduce the detection error.

As shown in FIG. 2, the transmitter module 11 further includes a light source collimating module 113 located between the small aperture stop 112 and the near-infrared light transmitter 111. The light source collimating module 113 is configured to collimate the near-infrared light transmitted by the near-infrared light transmitter 111 into parallel light rays, and then form a near-infrared aperture spot through the small aperture stop 112. The light source collimating module 113 includes a concave lens 114 and a convex lens 115. The concave lens 114 and the convex lens 115 are sequentially arranged in the path of the near-infrared light transmitted by the near-infrared light transmitter 111, and the concave lens 114 is arranged close to the near-infrared light transmitter 111. Therein, the concave lens 114 and the convex lens 115 use materials with a higher near-infrared transmittance, and a near-infrared antireflection film coated with double sides thereof.

Obviously, the light source collimation module 113 is only more effective for the present disclosure and may be omitted in some embodiments.

As shown in FIG. 2, the receive module 12 further includes a focusing module 122 for focusing the reflected near-infrared aperture spot before the near-infrared aperture spot reflected by the wearer's eye reaches the near-infrared receiver 121 .

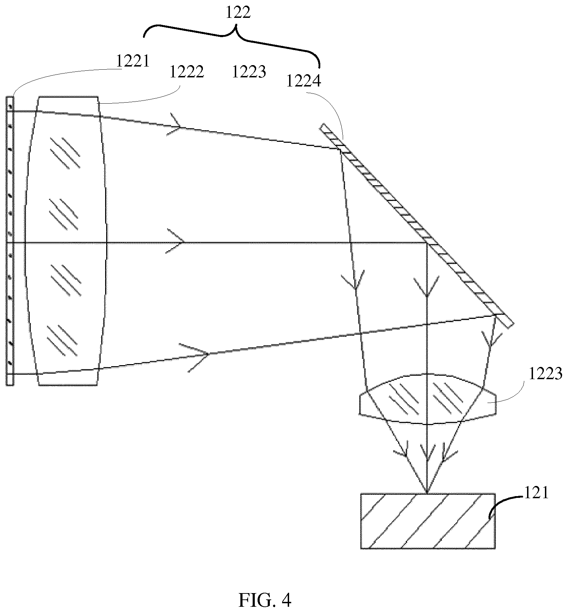

Referring to FIG. 4, the focusing module 122 includes a filter 1221, two convex lenses 1222, 1223, and a near-infrared total reflection mirror 1224. The filter 1221, the convex lens 1222, the near-infrared total reflection mirror 1224, and the convex lens 1223 are sequentially arranged in the propagation path from the near-infrared aperture spot reflected by the wearer's eye to the near-infrared receiver 121.

The filter 1221 is a narrow-band filter, which only allows the light of the wavelength of the near-infrared light transmitted by the near-infrared light transmitter 111 to pass through. The half bandwidth is less than 20 nm, and other wavelengths are cut off deeply to prevent other wavelengths from entering the system to interfere with the test result. The convex lens 1222 is configured to focus the near-infrared light of the filter 1221 for the first time. The near-infrared total reflection mirror 1224 is configured to reflect the small aperture stop of the first focusing near-infrared light through the convex lens 1222 to the convex lens 1223, and then pass through the convex lens 1223 to perform second focusing and then send it to the near-infrared receiver 121.

Obviously, the focusing module 122 is only more effective for the present disclosure and may be omitted in some embodiments.

As shown in FIG. 2, the display module 30 includes a display 31 and an optical module 302. The display 31 is configured to generate display images. The optical module 302 is configured to project the display images to the direction of exit pupil in a preset light path. The optical module 302 includes a second transflective element 32. The second transflective element 32 is located directly in front of the wearer's eye and forms a 45° angle with the light of the display images generated by the display 31. The second transflective element 32 is configured to reflect the display images to the wearer's eye so that the wearer can view the display images. The display 31 is a micro display for providing a display source including the display images. The display source forms an enlarged virtual image through a specific optical lens group (not labeled in the figure) and projects to the wearer's eye in a preset path. In this embodiment, the preset path is a path reflected by the second transflective element 32 to the wearer's eye. It can be understood that, in other embodiments, the position of the display 31 and the angle of the second transflective element 32 with respect to the display 31 can be flexibly set as long as the second transflective element 32 projects the light of the display source of the display 31 to the direction of the wearer's eye 200.

In this embodiment, the second transflective element 32 is located behind the diopter adjustment module 20, that is, relative to the concave lens 23 or the convex lens 24 of the diopter adjustment module 20, further away from the wearer's eye 200.

Therein, the first transflective element 13 is coated with a near-infrared transflective material on one side thereof facing the wearer's eye 200, and a visible light broadband antireflective material on the other side thereof. The incident angle is 45°.

As shown in FIG. 2 , the wearable display device 100 further includes a protective sheet 50. The protective sheet 50 is located on the side closest to the wearer's eye 200, and acts as a dustproof function for the entire optical system, and double sides are plated with visible light near-infrared broadband antireflection, the incident angle is 0°.

Therefore, the head mounted display device 100 of the present disclosure can automatically detect the diopter of the wearer's eye 200 and select to add the concave lens 23 or the convex lens 24 between the wearer's eyes 200 and the display images projected by the display module 30 when the diopter of the wearer's eye 200 is abnormal, so as to adjust the diopter of the wearer's eye 200 to be suitable for different people.

Therein, the head mounted display device 100 further includes other components, such as a sound output unit and a body frame. The sound output unit outputs a sound signal synchronized with the display images. The body frame is used to carry all the components described above. The improvement of the present disclosure is irrelevant and will not be described here.

The above is a preferred embodiment of the present disclosure, and it should be noted that those skilled in the art may make some improvements and modifications without departing from the principle of the present disclosure, and these improvements and modifications are also the protection scope of the present disclosure.

## Claims

1. A head mounted display device, comprising a display module, configured to project display images to a direction of an exit pupil along a preset light path, wherein, the head mounted display device further comprises:
a diopter detection module, configured to detect a parameter of the direction of exit pupil reflecting a diopter;
a diopter adjustment module, configured to adjust a focal length of the path; and
a controller, configured to determine whether or not the diopter standard threshold is met according to the parameter detected by the diopter detection module, when the diopter standard threshold is not met, the controller controls the diopter adjustment module to adjust, and further continuously acquires a current parameter detected by the diopter detection module during adjustment of the diopter adjustment module, until the current parameter detected by the diopter detection module is determined to meet the diopter standard threshold.

2. The head mounted display device according to claim 1, wherein the diopter detection module comprises a transmitter module and a receive module, the transmitter module is configured to transmit a near-infrared light to the direction of exit pupil, the receive module is configured to receive the near-infrared light reflected by the direction of exit pupil.

3. The head mounted display device according to claim 2, wherein the diopter adjustment module comprises a drive element, a first concave lens and/or a first convex lens, the controller is configured to determine whether or not the diopter standard threshold is met according to the near-infrared light received by the receive module, when the diopter standard threshold is not met, the concave lens or the convex lens is controlled to move closer to or move away from the exit pupil, until it is determined that the diopter standard threshold is met according to the near-infrared light reflected by the direction of exit pupil and received by the receive module.

4. The head mounted display device according to claim 3, wherein the first concave lens and/or the first convex lens are mounted on a shaft parallel to the direction of exit pupil and are rotated relative to the shaft under a drive of the drive element so as to be located on the corresponding exit pupil, and acted on an enable location of the optical path as a corrective element, or rotated to be at non-enable location to not act on the optical path; the first concave lens and/or the first convex lens moves along the shaft so as to move closer to or away from the exit pupil.

5. The head mounted display device according to claim 2, wherein the diopter detection module further comprises a first transflective element, located between the exit pupil and the receive module, a reflection plane of the first transflective element and a direction of the near-infrared light generated by the transmitter module are opposite to each other and are at an angle, the first transflective element is configured to reflect the near-infrared light transmitted by the transmitter module to the direction of the exit pupil and allows the near-infrared light reflected by the direction of the exit pupil to be transmitted to the receive module.

6. The head mounted display device according to claim 3, wherein, the transmitter module comprises a near-infrared light transmitter and a small aperture stop, the near-infrared light transmitter is configured to generate a near-infrared light and form a near-infrared aperture spot through the small aperture stop and project it to the direction of exit pupil, the receive module comprises a near-infrared receiver for receiving the near-infrared aperture spot reflected by the direction of exit pupil, the controller determines whether or not the diopter standard threshold is met based on a size of the near-infrared aperture spot received by the near-infrared receiver.

7. The head mounted display device according to claim 6, wherein, when the controller determines that a diameter of the near-infrared aperture spot is less than the diopter standard threshold, the drive unit is controlled to drive the concave lens to rotate to the enable location, and drive the concave lens to move relative to the direction of exit pupil until it is determined that the diopter standard threshold is met according to the near-infrared aperture spot continuously acquired; when the controller determines that the diameter of the near-infrared aperture spot is greater than the diopter standard threshold, the drive unit is controlled to drive the convex lens to rotate to the enable location initial position, and drive the concave lens to move relative to the direction of exit pupil until it is determined that the diopter standard threshold is met according to the near-infrared aperture spot continuously acquired.

8. The head mounted display device according to claim 7, wherein, the diopter standard threshold is 0.1mm.

9. The head mounted display device according to claim 6, wherein, the transmitter module further comprises a light source collimating module located between the small aperture stop and the near-infrared light transmitter, the light source collimation module is configured to collimate the near-infrared light transmitted by the near-infrared light transmitter into parallel light rays, and then form a near-infrared aperture spot through the small aperture stop.

10. The head mounted display device according to claim 9, wherein, the light source collimating module comprises a second concave lens and a second convex lens, the second concave lens and the second convex lens are sequentially arranged in a path of the near-infrared light transmitted by the near-infrared light transmitter, and the second concave lens is arranged close to the near-infrared light transmitter,, wherein, the second concave lens and the second convex lens use materials with a higher near-infrared transmittance, and a near-infrared antireflection film coated with double sides thereof.

11. The head mounted display device according to claim 6, wherein, the receive module further comprises a focusing module, the focusing module is configured for focusing the near-infrared aperture spot before the near-infrared aperture spot reflected by the direction of exit pupil reaches the near-infrared receiver.

12. The head mounted display device according to claim 11, wherein, the focusing module comprises a filter, a third convex lenses, a fourth convex lenses and a near-infrared total reflection mirror, wherein, the filter, the third convex lens, the near-infrared total reflection mirror, and the fourth convex lens are sequentially arranged in a propagation path from the near-infrared aperture spot reflected by the direction of exit pupil to the near-infrared receiver.

13. The head mounted display device according to claim 12, wherein, the filter is a narrow-band filter, for deeply cutting off other wavelengths, the third convex lens is configured to focus the near-infrared light passing through the filter, the near-infrared total reflection mirror is configured to reflect the small aperture stop of the near-infrared light focused by the third convex lens to the fourth convex lens, and then is focused by the fourth convex lens and then is received by the near-infrared receiver.

14. The head mounted display device according to claim 2, wherein, the display module comprises a display and an optical module, the display is configured to generate display images, the optical module is configured to project the display images to the direction of exit pupil along the preset light path.

15. The head mounted display device according to claim 14, wherein, the optical module comprises a second transflective element, the second transflective element is corresponding to the direction of exit pupil and forms a 45° angle with a light of the display images generated by the display, the second transflective element is configured to reflect the display images to the direction of exit pupil.

16. The head mounted display device according to claim 14, the second transflective element is located between the exit pupil and the receive module.

17. The head mounted display device according to claim 5, wherein, the first transflective element is coated with a near-infrared transflective material on one side thereof facing the exit pupil, and a visible light broadband antireflective material on other side thereof.

18. The head mounted display device according to claim 15, wherein, the second transflective element is coated with a visible light antireflective material on one side thereof facing the exit pupil and a visible light transparent material on other side thereof, an incident angle that the the light of the display images generated by the display relative to the second transflective element is 45°.

19. The head mounted display device according to claim 1, further comprising a protection sheet, the protection sheet is located close to a position of the exit pupil and serves as a dustproof function for an entire light path system, and coated with a visible-light broadband infrared light with double sides to increase penetrations.
